# EUROPÄISCHE PATENTANMELDUNG

(11) **EP 0 881 231 A2**
(43) Veröffentlichungstag der Anmeldung: **02.12.1998**
(21) Anmeldenummer: 98104367.2
(22) Anmeldetag: 30.06.1992
(51) Int. Cl.: C07K 14/00

(54) **HCV Peptidantigene und Verfahren zur Bestimmung von HCV**

(30) Priorität: 04.07.1991 DE 4122160; 14.12.1991 DE 4141304; 21.03.1992 DE 4209215
(62) Teilanmeldung aus: 92913343.7
(71) Anmelder: BOEHRINGER MANNHEIM GMBH, 68298 Mannheim (DE)
(72) Erfinder: Seidel, Christoph, Dr., 82362 Weilheim (DE); Ehrlich-Weinreich, Gertraud, Dr., 82327 Tutzing (DE); Bayer, Hubert, Dr., 69469 Weinheim (DE); Wienhues-Thelen, Uraula-Henrike, Dr., 82152 Krailling (DE); Ihlenfeldt, Hans-Georg, Dr., 82393 Iffeldorf (DE); Jung, Günther-Gerhard, Prof. Dr., 72076 Tübingen (DE)

(57) **Zusammenfassung**

Es werden neue HCV Peptidantigene, die Teilsequenzen des C-100-3 und env/core mit C-Bereichs darstellen, beschrieben. Diese Peptidantigene sind zur Bestimmung von HCV-Antikörpern als Immunogene zur Herstellung von Antikörpern gegen HCV und als Vakzine zur Herstellung von Impfstoffen gegen HCV geeignet.

## Beschreibung

Die Erfindung betrifft neue HCV Peptidantigene, ein Verfahren zur Herstellung dieser Peptidantigene, sowie ein Verfahren zur Bestimmung von HCV unter Verwendung der Peptidantigene.

Das Vorkommen von Virushepatitis in Abwesenheit serologischer Marker von bisher bekannten hepatotropen Agenzien (z.B. Hepatitis-A-Virus, Hepatitis-B-Virus, Hepatitis-Δ-Virus, Cytomegalie-Virus und Epstein-Barr-Virus) wird als Non-A, Non-B-Hepatitis (NANB-Hepatitis) bezeichnet. NANB-Hepatitis wird wiederum unterteilt in parenteral und sporadisch übertragene Non-A, Non-B-Hepatitis und enteral übertragene Non-A, Non-B-Hepatitis. Für die parenteral und sporadisch übertragene NANB-Hepatitis wurde vor kurzem das ursächliche Agenz, das Hepatitis-C-Virus (HCV) isoliert (Choo Q.-L. et. al. Science 244 (1989) 359 - 362 und Kino, G. et. al. Science 244 (1989) 362 - 364).

HCV ist weltweit eine wichtige Ursache von NANB-Hepatitis und wird durch kontaminiertes Blut oder Blutprodukte, Blutübertragungen oder durch engen persönlichen Kontakt übertragen.

Die Aminosäuresequenz der HCV-Virusproteine ist aus EP-A 0 318 216, EP-A 0 363 025, EPA 388 232 und EP-A 0 396 748 bekannt. Das Genom des HCV hat eine Länge von 10,862 nt. Die durch Translation hervorgehenden Proteine besitzen eine Gesamtlänge von ca. 3000 Aminosäuren. Die Proteine lassen sich in Strukturproteine (Hülle- und Kernproteine) und Nicht-Strukturproteine (NS1 - NS5) einteilen.

Die Bestimmung von HCV erfolgt zweckmäßig dadurch, daß durch immunologische Tests Antikörper gegen in Körperflüssigkeiten HCV nachgewiesen werden. Für derartige immunologische Tests werden deshalb Bindepartner für Anti-HCV-Antikörper benötigt.

So ist es bekannt, in immunologischen Tests beispielsweise das nicht-strukturelle C 100-3-HCV-Protein als Bindepartner zu verwenden (Tests von ABBOTT LABORATORIES, USA und ORTHO DIAGNOSTIC SYSTEMS ING., USA; Science 244 (1989) 359 - 364; Van der Poel C. L. et. al. Lancet 337(1991)317; Alter H.J. J. Gastroent. Hepatol. (suppl.) 1990, 78).

Nachteil dieser Tests ist, daß als Antigen ein rekombinantes Protein verwendet wird. Proteine sind wegen ihrer Denaturierungsanfälligkeit und damit verringerter Löslichkeit und Funktion in diagnostischen Tests schwer zu handhaben. Auch die Größe des Meßsignals ist aufgrund der niedrigen Epitopdichte auf einem Protein geringer als bei einem Test, bei dem ein kurzkettiges Peptidantigen als Bindepartner des Antikörpers verwendet wird. Weiter können bei Verwendung von Proteinen oder langkettigen Peptiden als Antigene in einem immunologischen Test vermehrt Kreuzreaktivitäten und unspezifische Bindungen von Antikörpern auftreten. Reaktionen mit Proteinen sind zudem oft diffusionskontrolliert, was den erwünschten kurzen Zeiten für immunologische Tests entgegensteht. Außerdem ist die Herstellung von für die Diagnostik einsetzbarem Protein in ausreichender Menge und Qualität zeitraubend und kostenintensiv. Peptide sind durch Synthese leicht zugänglich und sind definierte Moleküle.

Es ist demzufolge vorteilhaft, in einem immunologischen Test auf Anti-HCV-Antikörper möglichst kurzkettige Peptidantigene, die nur Ausschnitte der gesamten Proteine darstellen, zu verwenden. Ein derartiges immunologisches Verfahren ist von Okamoto (Japan J. Exp. Met. 60 (1990.) 223 - 234) beschrieben. Es hat sich jedoch gezeigt, daß das dort beschriebene kurzkettige Peptidantigen (Sequenz 9), welches aus der core-Region stammt, nicht ausreichend sensitiv für HCV ist.

Aufgabe der vorliegenden Erfindung ist es deshalb, Peptidantigene zur Verfügung zu stellen, die spezifisch für Anti-HCV-Antikörper sind und für immunologische Tests auf Anti-HCV-Antikörper geeignet sind.

Diese Aufgabe wird gelöst durch die Peptidantigene der Sequenzen 
1: SerGlyLysProAlaIleIleProAspArgGluValLeuTyrArgGluPheAsp
2: GluCysSerGlnHisLeuProTyrIleGluGlnGlyMetMetLeuAlaGluGlnPhe-
LysGlnLysAlaLeuGlyLeuLeuGlnThrAlaSerArgGln
3: AlaValGlnThrAsnTrpGlnLysLeuGluThrPheTrpAlaLysHisMetTrpAsn
4: AsnProLysProGlnLysLysAsnLysArgAsnThrAsnArgArg
5: AsnProLysProGlnArgLysThrLysArgAsnThrAsnArgArg
6: ProGlnAspValLysPheProGlyGlyGlyGlnIleValGlyGlyVal
7: ProArgGlySerArgProSerTrpGlyProThrAspProArgArg
8: GlnLeuPheThrPheSerProArgArgHisTrpThrThrGlnGlyCysAsnCys-
SerIleTyrProGlyHisIleThrGlyHisArgMetAlaTrpAspMetMetMet-
AsnTrpSerProThrThrAlaLeuValMetAla
10:GlnLysLysAlaAlaArgAsnThrAsnArgArg
11:HisTrpThrThrGlnGlySerAsnSerSerIleTyrProGlyHis
12:SerSerIleTyrProGlyHisIleThrGlyHisArgMetAlaTrpAspMetMet
13:ProGluGlyArgThrTrpAlaGlnProGlyTyrProTrpProLeuTyr
 oder Peptidantigene, die Teilsequenzen dieser Peptidantigene von mindestens vier, vorzugsweise von mindestens sieben, Aminosäuren Länge darstellen.

Geeignete Teilsequenzen sind in den Sequenzprotokollen dargestellt und mit Buchstaben/Zahl-Kombinationen (z.B. 6 a, 2 b) bezeichnet.

Besonders bevorzugte Teilsequenzen sind:

### aus Sequenz 2:

GluCysSerGlnHisLeuProTyrIleGluGlnGlyMetMetLeu
(Sequenz 2a)
MetMetLeuAlaGluGlnPheLysGlnLysAlaLeuGlyLeuLeuGlnThrAla
(Sequenz 2b)
MetMetLeuAlaGluGlnPheLysGlnLysAlaLeuGlyLeuLeuGlnThrAlaSerArg
Gln (Sequenz 2c)

HisLeuProTyrIleGlu (Sequenz 2d)

Ser Gln His Leu Pro Tyr Ile Glu Gln (Sequenz 2e)

Lys Ala Leu Gly Leu Leu Gln (Sequenz 2f)

Gln Lys Ala Leu Gly Leu Leu Gln Thr (Sequenz 2g)

### aus Sequenz 4:

Lys Asn Lys Arg Asn Thr Asn Arg Arg (Sequenz 4a)

### aus Sequenz 6:

ProGlnAspValLysPheProGlyGlyGlyGlnIle (Sequenz 6a)
Lys Phe Pro Gly Gly Gly Gln Ile Phe (Sequenz 6b)
Lys Phe Pro Gly Gly Gly Gln Ile Val (Sequenz 6d)
Gln Asp Val Lys Phe Pro Gly Gly Gly (Sequenz 6e)

Besonders bevorzugt werden Teiisequenzen, deren Länge maximal 9 Aminosäuren beträgt. Insbesondere sind dies die Sequenzen 6b, 6d, 6e, 2e, 2f, 2d, 2g, 4a.

Die Peptidantigene mit den Sequenzen 1 - 3 sind im C 100-3-Bereich der HCV-Proteine und die Peptidantigene mit den Sequenzen 4 - 8, 10 - 13 im env/core-Bereich der HCV-Proteine enthalten. Die erfindungsgemäßen Peptidantigene mit den Sequenzen 1 - 8, 10 - 13 und das Peptidantigen der Sequenz 9 
(ArgGlyProArgLeuGlyValArgAlaThrArgLysThrSerGluArgSerGln-
ProArgGlyArgArgGlnProIleProLysAlaArgArgProGluGlyArgThr-
TrpAlaGlnProGlyTyrProTrpPro, Okamoto loc. cit)
 sind durch die Sequenzprotokolle SEQ ID NO: 1 - 32 beschrieben.

Die Durchführung eines Anti-HCV-Antikörper-Nachweis erfolgt nach den dem Fachmann geläufigen Methoden. Ein weiterer Gegenstand der Erfindung ist deshalb ein Verfahren zur BeStimmung von HCV-Antikörpern, welches dadurch gekennzeichnet ist, daß die Probe mit einer Kombination von mindestens zwei Peptidantigenen aus der Gruppe der Sequenzen 1 - 13 oder Peptid-antigenen, die Teilsequenzen dieser Peptidantigene von mindestens vier, vorzugsweise von mindestens 7, Aminosäuren Länge darstellen, inkubiert und unter Bedingungen, die die Bildung eines Antikörper-Antigenkomplexes ermöglichen, die Menge der an das Peptidantigen gebundenen Anti-HCV-Antikörper bestimmt wird.

Erfindungsgemäß wird eine Kombination von mindestens zwei der erfindungsgemäßen Peptidantigene oder Teilsequenzen davon verwendet. Besonders bevorzugt ist es, die Peptidantigene der Sequenzen 1 - 3 oder Teilsequenzen davon mit mindestens einem Peptidantigen aus der Gruppe der Sequenzen 4 - 13 oder Teilsequenzen davon zu kombinieren.

Geeignete Teilsequenzen von Sequenz 9 sind: 
ArgGlyProArgLeuGlyValArgAlaThrArgLysThrSerGlnArgSerGlnProArg
Gly (Sequenz 9a)

SerGlnProArgGlyArgArgGlnProIleProLysAlaArgArgProGluGlyArgThr
(Sequenz 9b).
 Lys Ala Arg Arg Pro Glu Gly Arg Thr Trp Ala Gln Pro Gly Tyr (Sequenz 9c)

Die Kombination der Antigene kann beispielsweise dadurch erfolgen, daß mehrere einzelne Peptidantigene verwendet werden oder daß Peptidantigene kovalent, zweckmäßig über eine Aminosäurenbrücke, die sich von natürlicherweise in HCV-Proteinen vorkommenden Aminosäuren-Sequenzfolgen unterscheidet oder einen Peptid-Linker, aneinander gebunden sind.

Besonders bevorzugt sind Kombinationen der Antigene:
Sequenz 2b, 4 und 6
Sequenz 2b, 2c, 4 und 6
Sequenz 2a, 2b, 2c, 4 und 6
Sequenz 2a, 2b, 2c, 4, 6, 9a und 9b
Sequenz 2a, 2b, 4, 6, 9a und 3
Sequenz 2a, 2b, 4, 6 und 9a
Sequenz 2e, 2g, 4a, 6d, 6e
Sequenz 2d, 2f, 4a, 6c, 9c
Sequenz 11, 12, 8a

In Kombinationen werden die Antigene bevorzugt in etwa gleichen molaren Mengen verwendet.

Die Kombination der Antigene mit den Sequenzen 11, 12, 8a ist besonders geeignet zur Erkennung von Seren von Patienten, bei denen eine HCV-Infektion ausgeheilt ist (Rekonvaleszenzseren).

Vorzugsweise werden die Antigene einzeln, ohne kovalente Bindung aneinander, oder kovalent aneinander gebunden unter Verwendung eines Peptid-Linkers eingesetzt.

Auf Grund der erhöhten Empfindlichkeit, die bei dem Infektionsparameter HCV notwendig ist, werden vorzugsweise zum Nachweis heterogene Immunoassays verwendet. Diese heterogenen Tests erlauben Waschschritte, die den Meßsignal-Hintergrund erheblich reduzieren wodurch die Empfindlichkeit gesteigert wird.

Beispielsweise kann die Bestimmung durch einen Radio-Immunoassay, Enzym-Immunoassay oder durch Immunfloureszenz erfolgen. Üblicherweise wird hierzu das Peptidantigen immobilisiert. Die Probe, welche auf Anti-HCV-Antikörper untersucht werden soll, wird zugegeben und die an das Antigen gebundenen Antikörper über einen markierten Anti-Human-Immunglobulin-Antikörper bestimmt. Die Immobilisierung des erfindungsgemäßen Peptidantigens kann adsorbtiv, kovalent oder über ein biologisches Bindungspaar wie Biotin/Streptavidin, Antikörper/Antigen, oder Zucker/Lektin erfolgen. Dabei wird das Peptidantigen an diesen Partner kovalent gebunden.

Die erfindungsgemäßen Peptidantigene können für Immunoassays vorzugsweise nach dem Fachmann geläufigen Methoden, beispielsweise an Kugeln (beads), Kunststoffröhrchen oder Mikrotiterplatten (bevorzugt Polystyrol oder Copolymere von Polystyrol), immobilisiert werden. Dies erfolgt vorzugsweise dadurch, daß das Peptidantigen unspezifisch an der Oberfläche adsorbiert oder kovalent an funktionalisierte oder aktivierte Oberflächen gebunden wird. Die unspezifische Adsorption kann dadurch verbessert werden, daß man das Peptidantigen mit einem Protein zu einem Konjugat verknüpft und dieses Konjugat zur Adsorption verwendet (vgl. z.B. EP-A 0 269 092). Die Bindung kann auch über einen immobilisierten Antikörper erfolgen. Dazu sollte das Peptidantigen so verändert werden, daß das Epitop nicht durch die Bindung des Antikörpers blockiert wird z.B. durch Bildung eines Peptid-Protein-Konjugats.

Die Konjugation des Peptidantigens an den Bindungspartner erfolgt vorzugsweise über einen Spacer. Dieser Spacer enthält zweckmäßig 10 - 50, vorzugsweise 10 - 30 Atome und ist ebenfalls bevorzugt ein im wesentlichen lineares Molekül. Beispiele hierfür sind Spacer aus Alkyl-, Polyether- oder Polyamidketten. In einer besonders bevorzugten Ausführungsform ist ein Peptidantigen einer Länge von 4 - 9 Aminosäuren über einen linearen Spacer von 10 - 30 Atomen an den Träger gebunden. Falls ein Spacer aus Aminosäuren verwendet werden soll, besteht dieser zweckmäßig aus Aminosäuren, die nicht der Sequenzfolge in unmittelbarer Umgebung des Peptidantigens im HCV-Gen entsprechen.

In einer bevorzugten Ausführungsform wird das erfindungsgemäße Peptidantigen kovalent an Biotin gebunden, wobei die Immobilisierung über eine Avidin/Streptavidin-Festphase erfolgt.

Ebenso geeignet sind Bestimmungsverfahren, bei denen die Detektion nicht über einen markierten Antikörper, sondern über ein markiertes, weiteres Peptidantigen Sequenzen 1 - 13 oder Teilsequenzen davon erfolgt.

Die Herstellung der erfindungsgemäßen Peptidantigene ist nach den dem Fachmann geläufigen Methoden zur Peptidsynthese möglich. Ein weiterer Gegenstand der Erfindung ist deshalb ein Verfahren zur Herstellung des erfindungsgemäßen Peptidantigens, welches darin besteht, daß die das C-terminale Ende bildende Aminosäure an einen Träger gebunden wird, vom C-terminalen Ende das Peptidantigen schrittweise aufgebaut und anschließend vom Träger abgespalten wird.

Im einzelnen wird dazu eine Aminosäure beispielsweise über ihre Carboxygruppe an ein unlösliches, leicht filtrierbares Polymer geknüpft, und dann vom C-terminalen Ende her die Peptidkette schrittweise aufgebaut. Zu diesem Zweck wird eine N-geschützte Aminosäure mit einer reaktiven Gruppierung des Kunstharzes zur Reaktion gebracht. Von der am Trägerpartikel kovalent verankerten Aminosäure wird die Nα-Schutzgruppe entfernt und das resultierende Aminoacylpolymer mit der nächsten N-geschützten Aminosäure umgesetzt. Von dem am Trägerharz kovalent gebundenen Dipeptid wird die Nα-Schutzgruppe entfernt und das resultierende Aminoacylpolymer mit der nächsten N-geschützten Aminosäure umgesetzt. Alle überschüssigen Reagentien und Beiprodukte werden durch einfaches Filtrieren entfernt.Ist die gewünschte Peptidsequenz auf diese Weise hergestellt, wird die kovalente Bindung zwischen der C-terminalen Aminosäure und der Ankergruppierung des polymeren Trägers gespalten. Der unlösliche Träger wird durch einfache Filtration von dem nun in Lösung befindlichen Peptid entfernt. Das Peptid wird mittels chromatographischer Methoden gereinigt.

Beispielsweise können die erfindungsgemäßen Peptidantigene nach Merrifield, JACS 85(1964)2146 hergestellt werden. Eine gegebenenfalls erforderliche Biotinylierung kann beispielsweise nach PNAS USA 80 (1983) 4045 erfolgen. Ein bevorzugtes Biotinylierungsmittel hierfür ist Biotinylaminocapronsäure-N-hydroxysuccinimidester.

Ein bevorzugtes Verfahren zur Herstellung von biotinylierten Peptidantigenen ist die Einführung des Biotinrestes am N-Terminus während einer Festphasensynthese des Peptidantigens.

Dieses Verfahren wird bevorzugt dann verwendet, wenn das Peptidantigen mehrere ε-Lysin-Aminogruppen enthält, die nicht biotinyliert werden sollen. Dies ist beispielsweise dann der Fall, wenn man N-α-Fmoc-N-ε-biotinyl-aminocaproyl)lysin, N-α-Fmoc-N-ε-Biotinyilysin oder bei Biotinylierung der N-terminalen Aminosäuren Biotin, Biotinylaminocapronsäure oder Dimethoxytritylbiotin mit einem Aktivierungsreagenz wie zum Beispiel Dicyclohexylcarbodiimid oder als Aktivester einsetzt.

In einer weiteren bevorzugten Ausführungsform wird ein Nachweisantikörper der beispielsweise gegen den Fc-Teil von humanem IgG gerichtet ist, immobilisiert. Vorzugsweise wird hierzu ein monoklonaler Antikörper verwendet. Das Peptidantigen befindet sich dann in Lösung. Der nachzuweisende Antikörper (Analyt) und auch alle anderen Antikörper der Probenflüssigkeit werden vom Wandantikörper gebunden. Der gebundene Antikörper kann dann den Analyten binden, der mit einem geeigneten Nachweissystem, z.B. kompetitiv mit einem Peptidantigen-Enzymkonjugat nachgewiesen werden kann.

Mit den erfindungsgemäßen Peptidantigenen ist es auch möglich, durch die dem Fachmann geläufigen Verfahren der Immunisierung Antikörper zu erhalten, mit denen das Virus selbst in einem immunologischen Test nachgewiesen werden kann.

Ein weiterer Gegenstand der Erfindung ist deshalb ein Verfahren zur Herstellung von Antikörpern, welches dadurch gekennzeichnet ist, daß ein Säugetier mit einem erfindungsgemäßen Peptid, welches gegebenenfalls an einen Träger gebunden ist, immunisiert wird und die Antikörper nach bekannten Verfahren z.B. aus dem Serum oder der Milz gewonnen werden.

In einer bevorzugten Ausführungsform werden B-Lymphozyten der immunisierten Tiere in Gegenwart von transformierenden Agenzien mit einer geeigneten Zellinie fusioniert, die Zellinie, welche die gewünschten Antikörper produziert, kloniert und kultiviert und aus den Zellen oder dem Kulturüberstand die monoklonalen Antikörper gewonnen.

Mit diesem Antikörper ist es möglich, HCV-Viren direkt zu bestimmen. Ein weiterer Gegenstand der Erfindung ist deshalb ein Verfahren zur Bestimmung von HCV-Viren, welches dadurch gekennzeichnet ist, daß die Probe mit einem erfindungsgemäßen Antikörper unter Bedingungen, die eine Antigen-Antikörperkomplexbildung erlauben, inkubiert, und die Menge des gebildeten Antikörper-Antigenkomplexes bestimmt wird.

Ein weiterer Gegenstand der Erfindung ist ein Verfahren zur Herstellung von Impfstoffen unter Verwendung der erfindungsgemäßen Peptidantigene sowie ein Vakzin zur Behandlung von HCV-Infektionen, enthaltend als Immunogen ein gegebenenfalls trägergebundenes Peptidantigen der Sequenzen 1 - 8, 10 - 13 oder Teilsequenzen davon oder mindestens zwei Peptidantigene der Sequenzen 1 - 13 oder Teilsequenzen davon in einer pharmakologisch effektiven Dosis und in einer pharmazeutisch akzeptablen Formulierung.

Die Herstellung dieser Impfstoffe kann nach den bekannten Methoden durchgeführt werden. Bevorzugt werden jedoch die Peptidantigene zunächst lyophilisiert und anschließend, ggf. unter Zusatz von Hilfsstoffen, suspendiert.

Die Impfung mit dem erfindungsgemäßen Vakzin oder Vakzinkombinationen kann durch die dem Fachmann geläufigen Methoden erfolgen, beispielsweise intradermal, intramuskulär, intraperitoneal, intravenös, subkutan und intranasal.

Zur intramuskulären oder subkutanen Gabe kann das Vakzin beispielsweise in physiologischer Kochsalzlösung suspendiert sein. Zur intranasalen oder intraoccularen Applikation kann das Vakzin, beispielsweise in Form eines Sprays oder einer wäßrigen Lösung angewendet werden. Für lokale, beispielsweise orale Gabe ist es häufig erforderlich, die Immunogene zeitweise gegen Inaktivierung zu schützen, beispielsweise gegen proteolytische Enzyme in der Mundhöhle oder im Magen. Ein derartiger vorübergehender Schutz kann beispielsweise durch Verkapselung der Immunogene erfolgen. Diese Verkapselung kann beispielsweise durch Überziehen mit einem Schutzmittel (Mikroverkapselung) oder bei Einbettung einer Vielzahl von erfindungsgemäßen Immunogenen in einen schützenden Träger (Makroverkapselung) erfolgen.

Das Verkapselungsmaterial kann semipermiabel sein oder beim Einbringen in den menschlichen oder tierischen Körper semipermeabel werden. Üblicherweise wird für die Verkapselung eine biologisch abbaubare Substanz als Träger verwendet.

Die nachfolgenden Beispiele und Sequenzprotokolle erläutern die Erfindung weiter.

In den Sequenzprotokollen bedeuten:

| Sequenz | SEQ ID NO |
|---|---|
| 1 | 1 |
| 2 | 2 |
| 2 a | 3 |
| 2 b | 4 |
| 2 c | 5 |
| 2 d | 6 |
| 2 e | 7 |
| 2 f | 8 |
| 2 g | 9 |
| 2 h | 10 |
| 3 | 11 |
| 4 | 12 |
| 4 a | 13 |
| 4 b | 14 |
| 5 | 15 |
| 6 | 16 |
| 6 a | 17 |
| 6 b | 18 |
| 6 c | 19 |
| 6 d | 20 |
| 6 e | 21 |
| 7 | 22 |
| 8 | 23 |
| 8 a | 24 |
| 9 | 25 |
| 9 a | 26 |
| 9 b | 27 |
| 9 c | 28 |
| 10 | 29 |
| 11 | 30 |
| 12 | 31 |
| 13 | 32 |

### Beispiel 1

### Synthese von H-ProArgGlySerArgProSerTrpGlyProThrAspProArgArg-OH

Das Peptid wurde mittels Fmoc(Fluorenyloxycarbonyl)-Festphasensynthese hergestellt. Die Reaktionen wurden an einem Labortec (Schweiz) SP 640 Peptidsynthesizer durchgeführt. Die Kupplungsreaktionen wurden bezüglich Fmoc-Aminosäure-Derivat mit 2.4 Äquivalenten Dicyclohexylcarbodiimid und 2.2 Äquivalenten N-Hydroxybenzotriazol während 90 Minuten durchgeführt. Als Reaktionsmedium kam Dimethylformamid zum Einsatz. Die Fmoc-Gruppe wurde mittels 20 % Piperiden in DMF in 10 und 20 Minuten abgespalten. 2.0 Äquivalente von den folgenden Aminosäure-Derivaten kamen zum Einsatz: Pro, Arg(mit PMC (Pentamethylchroman)-Schutzgruppe), Gly, Ser(mit tert.-Butyl-Schutzgruppe), Trp, Thr(mit tert.-Butyl-Schutzgruppe), Asp(mit tert.-Butylester-Schutzgruppe). Die Kupplungsreaktionen wurden mit der Hälfte der Reagentien wiederholt. Der Kupplungserfolg wurde mittels Kaiser-Test (Anal. Biochemistry 34(1970)595) geprüft, die Harzbeladung wurde mittels UV-Absorption der freigesetzten Fulven-Gruppe nach jeder Piperidin-Spaltung ermittelt. Das Peptid wurde an 5 g Wang-Harz (Polystyrol/1 % Divinylbenzol) mit einer Ladung von 0.50 mMol/g synthetisiert (JACS 95(1973)1328). Nach der Synthese betrug der Beladungsgrad noch 0.39 mMol/g.

Die Freisetzung des Peptids wurde mit 200 ml Trifluoressigsäure, 200 ml Dichlormethan, 10 ml Ethandithiol, 10 ml m-Kresol, 5 ml Ethylmethylsulfid und 5 ml Wasser in 30 Minuten bei Raumtemperatur. Die Abspaltlösung wurde mehrmals mit Toluol eingeengt, dann das Peptid mit Diethylether gefällt.

Zur Entfernung der Scavenger und anderer kleiner Moleküle wurde das Rohmaterial über eine Sephadex G10-Säule gereinigt. Es wurden nach Lyophilisieren 3.2 g Material einer Reinheit von 42 % (RP-HPLC) erhalten. Um das Material auf die Endreinheit von >95 % zu bringen, wurden 400 mg Peptid über eine präparative RP-HPLC-Säule (40mmx250mm) gefüllt mit C18-Material (5 Mikrometer, 300 Angström) und einem Wasser/Trifluoressigsäure-, Acetonitril/Trifluoressigsäure-Gradienten gereinigt. Es fielen nach Lyophilisation 118 mg 96.5 %iges (HPLC) weißes Material an. Die Identität des Materials wurde mittels FAB-MS geprüft.

### Beispiel 2

Zur Biotinylierung des Peptidantigens aus Beispiel 1 wurde ein Moläquivalent möglichst konzentriert (Löslichkeit ist von der Aminosäuresequenz abhängig) in Argon-gesättigtem Kaliumphosphatpuffer (0,1 mol/l, pH 8,0) gelöst und mit 3 Äquivalenten D-Biotinyl-ε-aminocapronsäure-N-hydroxysuccinimidester gelöst in Argon-gesättigtem Dimethylformamid (Lösung von 1 µmol Reagenz in 5 µl DMF) versetzt.

Man rührte das Reaktionsgemisch 2 Stunden bei Raumtemperatur unter Argon unter ständiger Kontrolle mittels analytischer RP-HPLC. Wenn < 5% Edukt vorhanden waren, wurde der Reaktionsansatz direkt auf eine präparative RP-HPLC-Säule gegeben und das Produktmaterial mittels einem 0.1% Trifluoressigsäure/Wasser- zu 0.1% Trifluoressigsäure/ Acetonitril-Gradienten (Steigung: 0% auf 100% in 90 Minuten) gereinigt. Das Produktmaterial wurde durch Einengen und Lyophilisation der Produktfraktionen erhalten. Die Ausbeuten lagen zwischen 40 % und 90%. Als Analytik dienten für die Reinheit HPLC, HPCE und TLC, für die Identität FAB-MS (Molpeak) und TLC mit spezifischen Anfärbereagentien (p-Dimethylaminozimtaldehyd auf Biotin) und Gehaltsbestimmung über Mikroanalyse (Nitrogen).

### Beispiel 3

HCV-Antikörper werden in einem 2-Schritt-Sandwich-Immunoassay bestimmt. Zum Nachweis werden die Reagentien mit folgender

### Zusammensetzung verwendet:

### Reagenz 1:

0.10 µg/ml (Peptidantigene 1,3,4,5,6) oder 0.25 µg/ml (Peptidantigene 2,4,7), biotinyliertes Peptidantigen oder eine
1:1 Mischung solcher Peptidantigene
40 mmol/l Phosphatpuffer pH 7.0
0.9 Gew.-% NaCl
10 Vol.-% Rinderserum

### Reagenz 2:

20 mU/ml eines Konjugats aus polyklonalem Antikörper gegen humanes Immunglobulin (Schaf) und Peroxidase
40 mmol/l Phosphatpuffer pH 7.0
0.05 Gew.-% Tween® 20
0.2% Rinderserumalbumin
0.2% Rinder-IgG

In einem mit Streptavidin beschichteten Polystyrolröhrchen (hergestellt nach Beispiel 1 von EP-A 0 344 578) werden 1 ml Reagenz 1 und 10 µl Probe eine Stunde bei Raumtemperatur inkubiert. Anschließend wird dreimal mit Leitungswasser gewaschen und mit 1 ml Reagenz 2 für eine Stunde bei Raumtemperatur inkubiert. Anschließend wird dreimal mit Leitungswasser gewaschen. Zur Nachweisreaktion werden 1 ml ABTS® (2,2'-Azino-di-[3-ethyl-benzthiazolinsulfonat(6)]-Diammoniumsalz, 1,9 mmol/l, in 100 mmol/l Phosphatcitratpuffer pH 4.4 mit 3.2 mmol/l Natrium-perborat) zugegeben. Nach 60 min wird die Extinktion bei 420 nm photometrisch gemessen. Die Ergebnisse zeigt Tabelle 1:

**Tabelle 1**

| Serum | Peptidantigene (Sequenz Nr.) | | | | | | | | | | |
|---|---|---|---|---|---|---|---|---|---|---|---|
| | 9 | 1 | 2 | 3 | 4 | 5 | 6 | 7 | 8 | 1+4 | 3+6 |
| 1 | + | - | + | + | + | + | + | - | + | + | + |
| 2 | - | - | - | - | + | + | + | - | - | + | + |
| 3 | - | + | - | - | - | - | + | - | - | + | + |
| 4 | + | - | + | + | + | - | + | + | + | + | + |
| 5 | - | - | + | - | + | - | - | - | + | + | + |
| 6 | + | - | + | + | + | + | + | - | + | + | + |
| 7 | + | + | + | - | + | - | + | + | + | + | + |

### Erläuterungen zu Tabelle 1:

- -/+:: negativ/positiv (Der Cutoff für ein positives Signal im ELISA ist definiert als die mittlere Extinktion bei 420 nm plus 3 Standardabweichungen eines Kollektivs von 10 negativen Kontrollseren. Die Proben wurden bei einer Probenverdünnung von 1:250 vermessen).

Serum 1 war negativ im Test im Ortho-HCV-Antikörper ELISA-Test-system von ORTHO DIAGNOSTIC SYSTEMS INC. jedoch positiv aufgrund des klinischen Bildes.

Die Seren 2 - 5 wurden nach dem Test von Ortho Laboratories, die Seren 6 und 7 mit dem ABBOTT HCV EIA, Kat.No. 3 A53-24, ABBOTT LABORATORIES INC. als positiv identifiziert.

Die Peptidantigene 1 - 6 wurden mit Dimethoxytrityl-Biotin festphasenbiotinyliert an der ε-Aminogruppe eines zusätzlich N-terminal eingeführten Lysins.

Die Peptidantigenmischungen 1 + 4 und 3 + 6 wurden in einem molaren Mischungsverhältnis von 1:1 eingesetzt.

### Beispiel 4

Es wurden weitere Seren mit Peptiden und Peptidmischungen in einem Zweischritt-Sandwich-Immunoassay auf mit Streptavidin beschichteten Mikrotiterplatten geprüft.

Die Durchführung der Bestimmung erfolgte weitgehend analog Beispiel 3. Dabei wurden folgende Reagentien verwendet:

### Reagenz 1:

50 ng Peptid (oder die in den Tabellenerklärungen genannten Mengen) in 100 µl Inkubationspuffer (40 mmol/l Phosphatpuffer, pH 7,0, 0,9 Gew.-% NaCl, 10 Vol.-% Rinderserum.

### Reagenz 2:

Konjugat aus polyklonalem Antikörper gegen humanes Immunglobulin (Schaf) und Peroxidase (Peroxidaseaktivität 20 mU/ml), 40 mmol/l Phosphatpuffer pH 7,0, 0,05 Gew.-% Tween® 20, 0,2 % Rinderserumalbumin, 0,2 % Rinder-IgG.

### Waschlösung

40 mmol/l Phosphatpuffer pH 7,0, 0,9 Gew.-% Natriumchlorid, 0,05 Gew.-% Tween® 20.

### Farbreagenz

10 mg ABTS®, 80 µl 0,4 % H₂O₂ in 10 ml Citrat-Phosphatpuffer (pH 4,4, 100 mmol/l).

Pro Napf (well) einer mit Streptavidin beschichteten Mikrotiterplatte werden Serum (1 : 10 verdünnt in 50 µl Inkubationspuffer und 100 µl Reagenz 1 zugegeben. Es wird eine Stunde bei Raumtemperatur inkubiert und anschließend fünfmal mit je 200 µl Waschlösung gewaschen. Es werden 150 µl Reagenz 2 zugegeben, eine Stunde bei Raumtemperatur inkubiert und dreimal mit je 200 µl Waschlösung gewaschen. Es werden 150 µl Farbreagenz zugegeben, eine Stunde bei Raumtemperatur inkubiert und die Extinktion bei 420 nm photometrisch gemessen.

Die Tabellen II, III, IV, V, VI und VII zeigen die Ergebnisse.

In den Tabellen bedeuten:

### Tabelle II:

- Ortho:: relative Größe des Meßsignals im Test von Ortho (vgl. Beispiel 3.
- leeres Feld:: Meßwert kleiner als zweifacher Leerwert oder identisch mit Leerwert (bestimmt mit biotinyliertem, mit HCV-Antikörpern nicht reaktivem Peptid (nonsens-Sequenz)).
- voller Kreis:: Meßwert dreifacher Leerwert oder größer bei 50 ng Peptid pro Napf.
- leerer Kreis:: Meßwert zweifacher Leerwert bei 50 ng Peptid pro Napf
- volles Quadrat:: Meßwert dreifacher Leerwert oder größer bei 250 ng Peptid pro Napf.
- leeres Quadrat:: Meßwert zweifacher Leerwert oder größer bei 250 ng Peptid pro Napf.
- * :: Negativkontrollen

### Tabelle III

- leeres Feld:: wie Tabelle II
- voller Kreis:: Meßwert vierfacher Leerwert oder größer bei 50 ng Peptid pro Napf.
- leerer Kreis:: Meßwert dreifacher Leerwert oder größer bei 50 ng Peptid pro Napf.
- n.t.:: Messung nicht durchgeführt
- 2a, 2b, 3, 4, 6:: Anstelle eines einzelnen Peptidantigens wurde in Reagenz 1 eine Mischung aus je 10 ng der genannten Peptide verwendet.
- *:: Negativkontrollen

### Tabelle IV

Die Bedeutung der Symbole entspricht den Angaben zu Tabelle II.

Die Peptidmischungen enthielten jeweils 50 ng der einzelnen Peptide.

### Beispiel 5:

### Tabellen V, VI und VII

Ergebnisse von Immunoassays analog Beispiel 3, wobei im Reagenz 1 folgende Peptidkonzentrationen eingesetzt wurden:

Bei Verwendung von mehreren Antigenen in Kombination werden die Einsatzmengen entsprechend der Anzahl der verschiedenen Antigene reduziert.

| | |
|---|---|
| Sequenz 2a | 50 µg/ml |
| Sequenz 2b | 50 µg/ml |
| Sequenz 2d | 100 µg/ml |
| Sequenz 2f | 100 µg/ml |
| Sequenz 2h | 100 µg/ml |
| Sequenz 4 | 400 µg/ml |
| Sequenz 4a | 350 µg/ml |
| Sequenz 4b | 250 µg/ml |
| Sequenz 4c | 300 µg/ml |
| Sequenz 6 | 350 µg/ml |
| Sequenz 6a | 350 µg/ml |
| Sequenz 6b | 350 µg/ml |
| Sequenz 6c | 250 µg/ml |
| Sequenz 6d | 300 µg/ml |
| Sequenz 8a | 900 µg/ml |
| Sequenz 9a | 350 µg/ml |
| Sequenz 9c | 350 µg/ml |
| Sequenz 11 | 300 µg/ml |
| Sequenz 12 | 550 µg/ml |

+/- : pos./neg. (Der Cutoff für ein positives Signal im Immunoassay wie Beispiel 3 ist definiert als die mittlere Extinktion bei 420 nm plus 2 Standardabweichungen eines Kollektivs von 6 negativen Kontrollseren. Die Proben sind mit Inkubationspuffer 1 : 100 verdünnt).

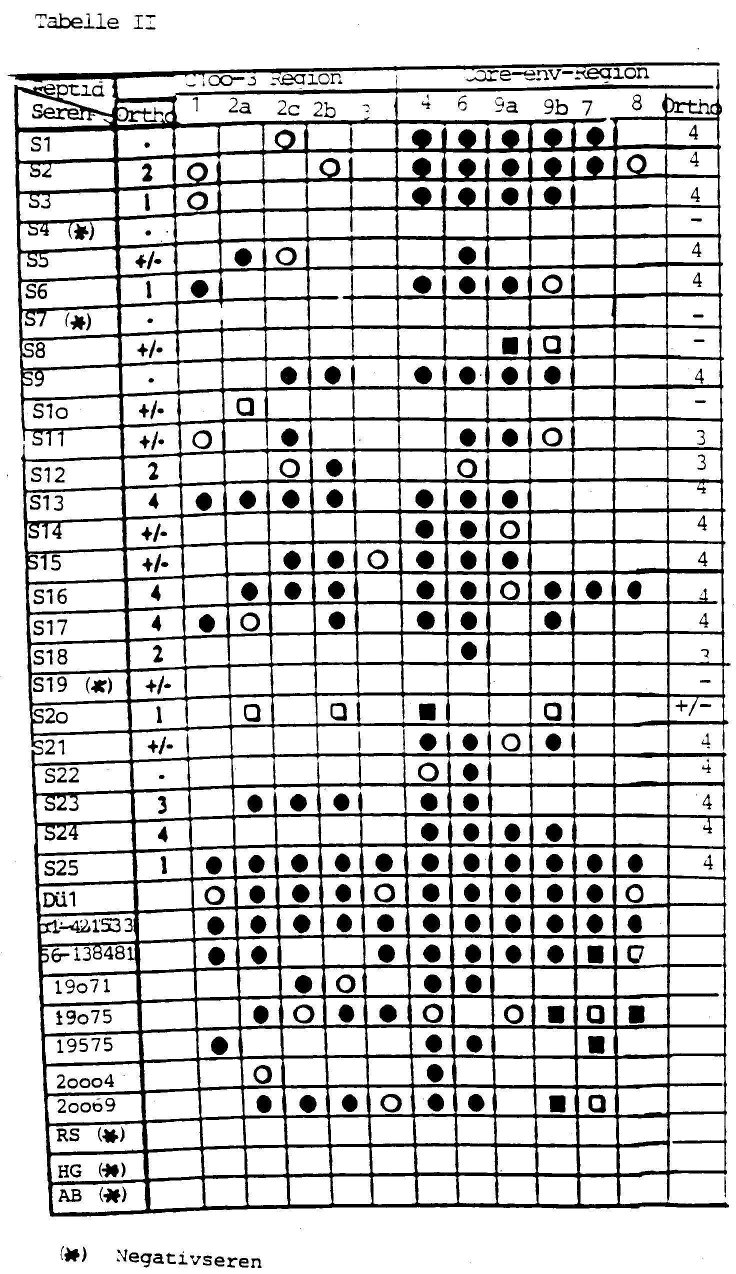

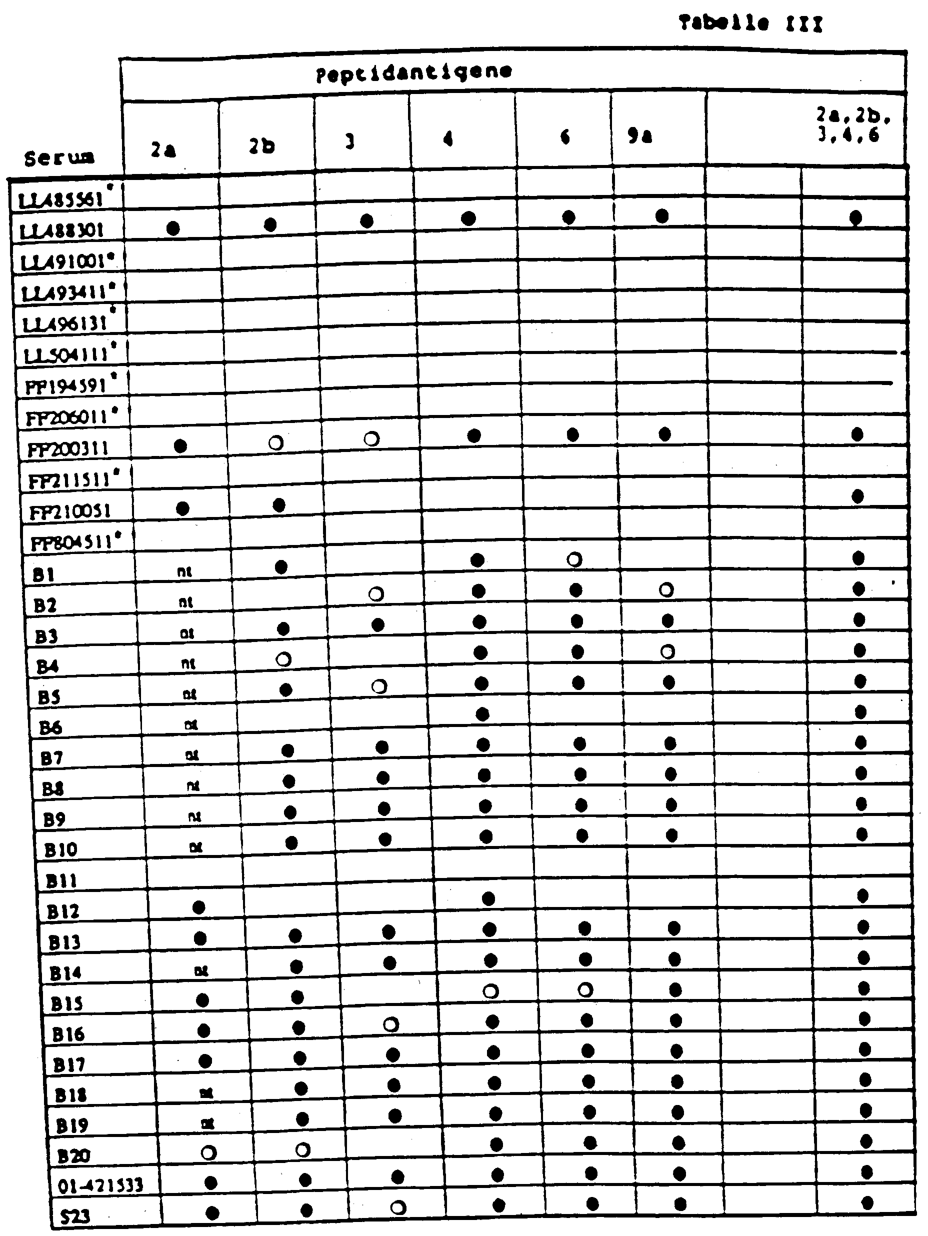

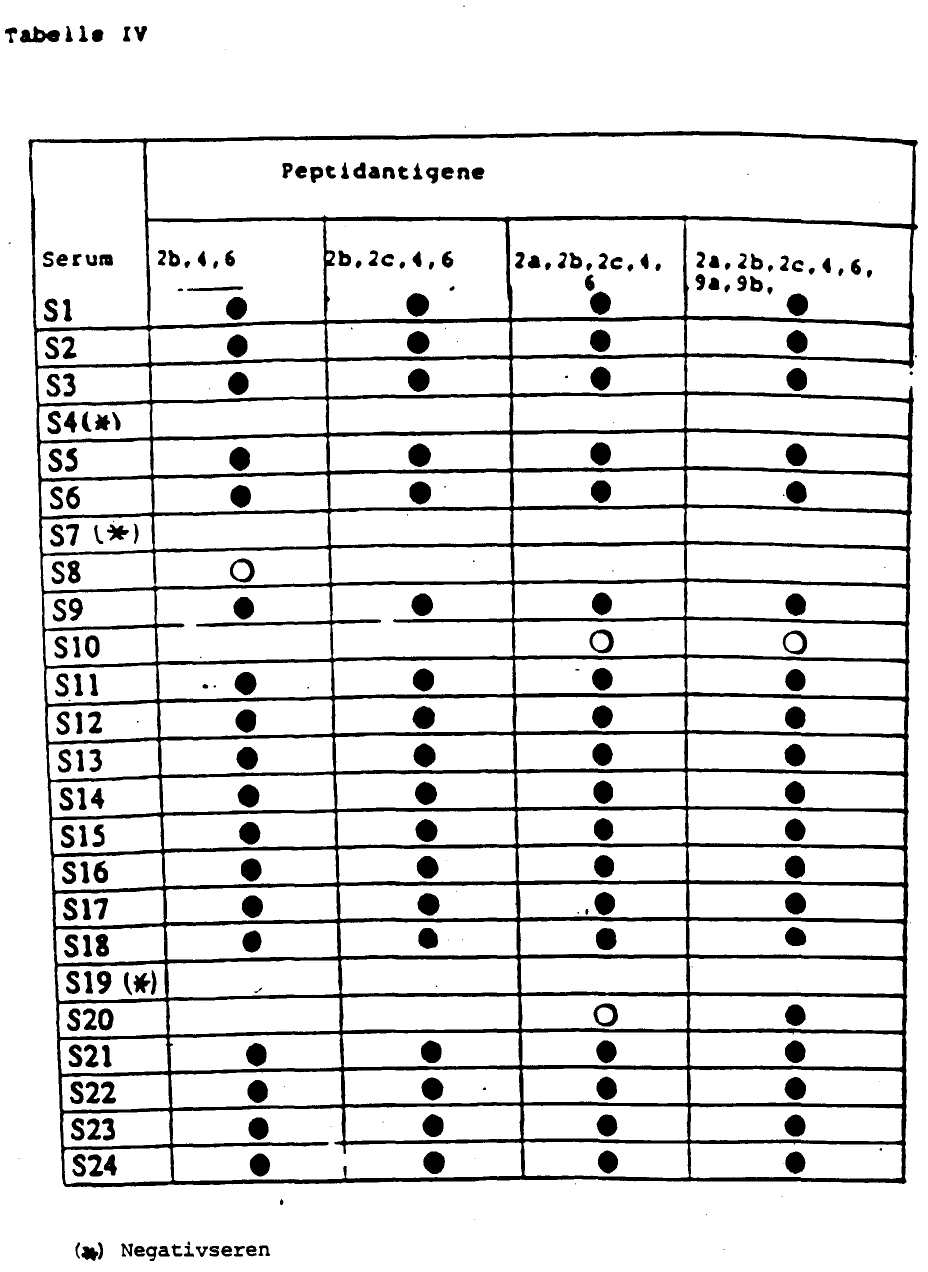

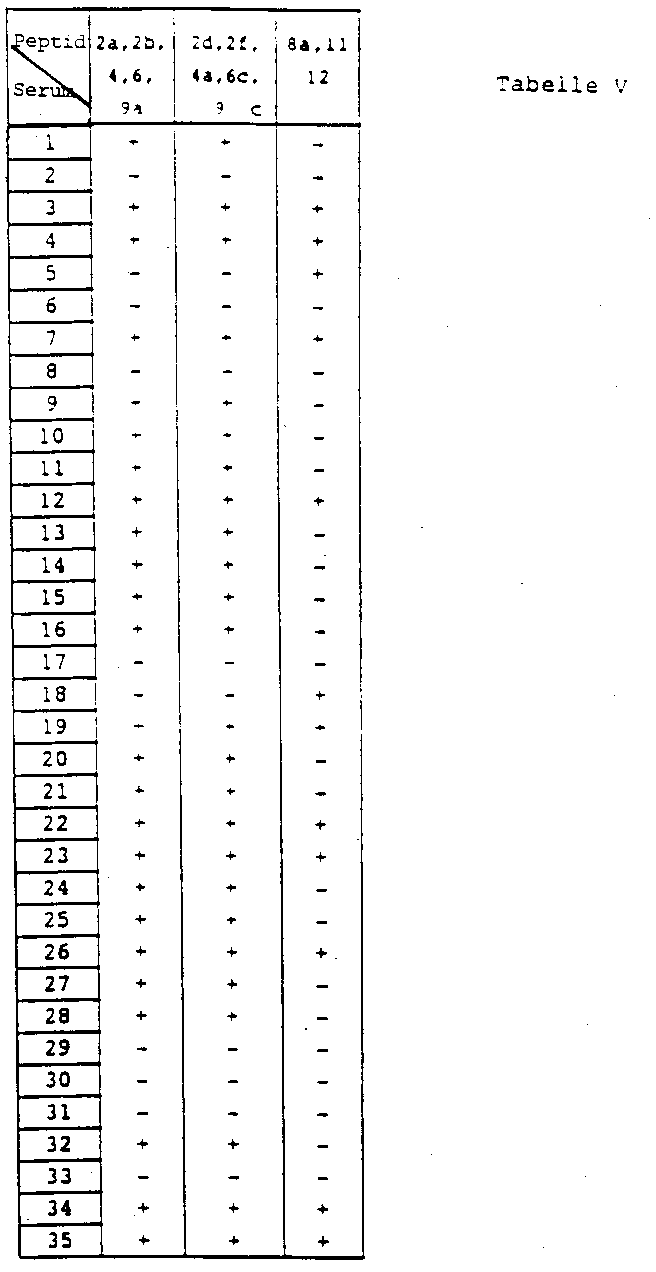

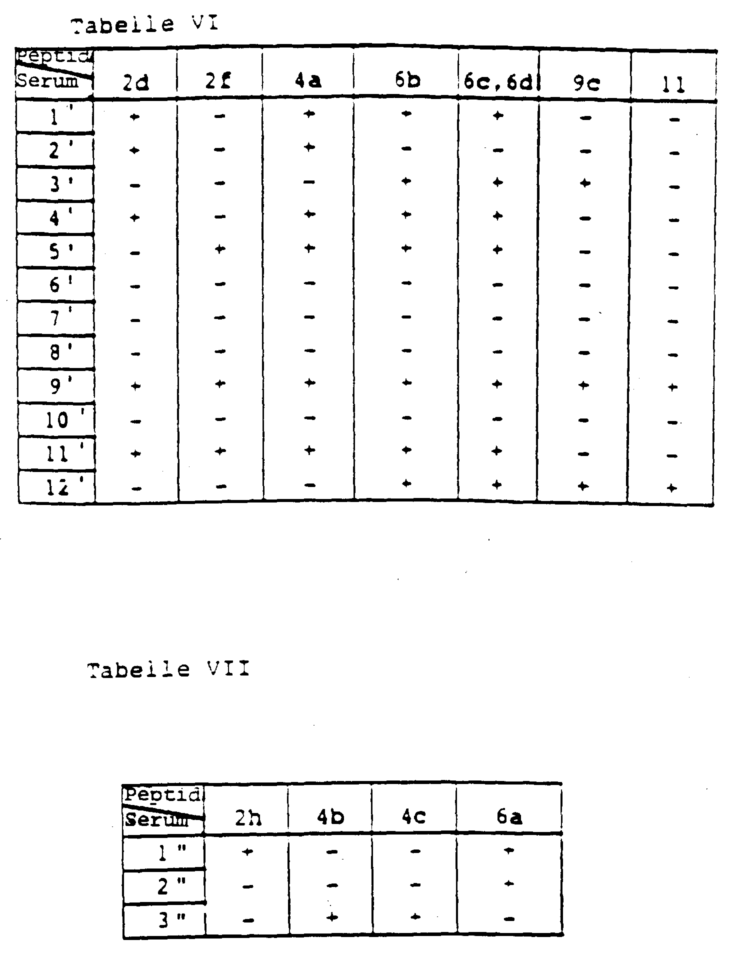

## Patentansprüche

1. Peptidantigene aus dem core-Bereich von HCV mit den Aminosäuresequenzen SEQ ID NO 12-15, 16-21, 22, 25-29, 32 oder Teilsequenzen davon mit einer Länge von mindestens 4 Aminosäuren.

2. Verfahren zur Herstellung von Peptidantigenen nach Anspruch 1, dadurch gekennzeichnet, daß die das C-terminale Ende bildende Aminosäure an einen Träger gebunden wird, vom C-terminalen das Peptidantigen schrittweise aufgebaut und anschließend vom Träger abgespalten wird.

3. Verfahren zur Bestimmung von HCV-Antikörpern, dadurch gekennzeichnet, daß die Probe mit mindestens einem Peptidantigen gemäß Anspruch 1 inkubiert wird und unter Bedingungen, die die Bildung eines Antigen-Antikörper-Komplexes ermöglichen, die Menge der an das Peptidantigen gebundenen HCV-Antikörper bestimmt wird.

4. Verwendung von Peptidantigenen aus dem core-Bereich von HCV gemäß Anspruch 1 zum immunologischen Nachweis von HCV-Antikörpern.

5. Verfahren zur Herstellung von Antikörpern gegen HCV-Antigene, dadurch gekennzeichnet, daß ein Säugetier mit einem gegebenenfalls trägergebundenen Peptidantigen gemäß Anspruch 1 immunisiert wird, polyklonale Antikörper gewonnen werden oder Zellen dieser Tiere, die Antikörper produzieren, zu Zellinien immortalisiert werden und aus diesen Zellinien monoklonale Antikörper gewonnen werden.

6. Verfahren zur Bestimmung von HCV-Viren, dadurch gekennzeichnet, daß die Probe mit einem Antikörper nach Anspruch 5 unter Bedingungen, die eine Antigen-Antikörper-Komplexbildung erlauben, inkubiert wird, und die Menge des gebildeten Antigen-Antikörper-Komplexes bestimmt wird.

7. Vakzin zur Behandlung von HCV-Infektionen, enthaltend ein gegebenenfalls trägergebundenes Peptidantigen gemäß Anspruch 1 als Immunogen in einer pharmakologisch effektiven Dosis und in einer pharmakologisch akzeptablen Formulierung.

8. Verfahren zur Herstellung von Impfstoffen unter Verwendung der Peptidantigene gemäß Anspruch 1.


SEQUENZPROTOKOLL

(1) ALLGEMEINE INFORMATION:

(i) ANMELDER:
(A) NAME: Boehringer Mannheim GmbH
(B) STRASSE: Sandhoferstr. 116
(C) ORT: Mannheim
(E) LAND: DE
(F) POSTLEITZAHL: 68298

(ii) ANMELDETITEL: HCV Peptidantigene und Verfahren zur Bestimmung von
HCV

(iii) ANZAHL DER SEQUENZEN: 32

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 18 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 1:

Ser Gly Lys Pro Ala Ile Ile Pro Asp Arg Glu Val Leu Tyr Arg Glu
1               5                   10                  15

Phe Asp

(2) INFORMATION ZU SEQ ID NO: 2:

(i) SEQUENZ CHARAKTERISTIKA:
(A) LÄNGE: 33 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 2:

Glu Cys Ser Gln His Leu Pro Tyr Ile Glu Gln Gly Met Met Leu Ala
1               5                   10                  15

Glu Gln Phe Lys Gln Lys Ala Leu Gly Leu Leu Gln Thr Ala Ser Arg
            20                  25                  30

Gln

(2) INFORMATION ZU SEQ ID NO: 3:

(i) SEQUENZ CHARAKTERISTIKA:
(A) LÄNGE: 15 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 3:

Glu Cys Ser Gln His Leu Pro Tyr Ile Glu Gln Gly Met Met Leu
1               5                   10                  15

(2) INFORMATION ZU SEQ ID NO: 4:

(i) SEQUENZ CHARAKTERISTIKA:
(A) LÄNGE: 18 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 4:

Met Met Leu Ala Glu Gln Phe Lys Gln Lys Ala Leu Gly Leu Leu Gln
1               5                   10                  15

Thr Ala

(2) I[?]FORMATION ZU SEQ ID NO: 5:

[?]i) SEQUENZ CHARAKTERISTIKA:
(A) LÄNGE: 21 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 5:

Met Met Leu Ala Glu Gln Phe Lys Gln Lys Ala Leu Gly Leu Leu Gln
1               5                   10                  15

Thr Ala Ser Arg Gln
            20

(2) INFORMATION ZU SEQ ID NO: 6:

(i) SEQUENZ CHARAKTERISTIKA:
(A) LÄNGE: 6 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 6:

His Leu Pro Tyr Ile Glu
1               5

(2) INFORMATION ZU SEQ ID NO: 7:

(i) SEQUENZ CHARAKTERISTIKA:
(A) LÄNGE: 9 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 7:

Ser Gln His Leu Pro Tyr Ile Glu Gln
1               5

(2) INFORMATION ZU SEQ ID NO: 8:

(i) SEQUENZ CHARAKTERISTIKA:
(A) LÄNGE: 7 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 8:

Lys Ala Leu Gly Leu Leu Gln
1               5

(2) INFORMATION ZU SEQ ID NO: 9:

(i) SEQUENZ CHARAKTERISTIKA:
(A) LÄNGE: 9 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 9:

Gln Lys Ala Leu Gly Leu Leu Gln Thr
1               5

(2) INFORMATION ZU SEQ ID NO: 10:

(i) SEQUENZ CHARAKTERISTIKA:
(A) LÄNGE: 10 Aminosäuren
(B) ART: Aminosäure
(D) TOPOLOGIE: linear

(ii) ART DES MOLEÜLS: Peptid

(xi) SEQUENZBESCHREIBUNG: SEQ ID NO: 10:

Gln Lys Ala Leu Gly Leu Leu Gln Thr Ala
1               5                   10

(2) INFORMATION ZU SEQ ID NO: 11:

(i) SEQUENZ CHARAKTERISTIKA:
(A) LÄNGE: 19 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 11:

Ala Val Gln Thr Asn Trp Gln Lys Leu Glu Thr Phe Trp Ala Lys His
1               5                   10                  15

Met Trp Asn

(2) INFORMATION ZU SEQ ID NO: 12:

(i) SEQUENZ CHARAKTERISTIKA:
(A) LÄNGE: 15 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 12:

Asn Pro Lys Pro Gln Lys Lys Asn Lys Arg Asn Thr Asn Arg Arg
1               5                   10                  15

(2) INFORMATION ZU SEQ ID NO: 13:

(i) SEQUENZ CHARAKTERISTIKA:
(A) LÄNGE: 9 Aminoäuren
(B) ART: Aminosäure
(D) TOPOLOGIE: linear

(ii) ART DES MOLEKÜLS: Peptid

(xi) SEQUENZBESCHREIBUNG: SEQ ID NO: 13:

Lys Asn Lys Arg Asn Thr Asn Arg Arg
1               5

(2) INFORMATION ZU SEQ ID NO: 14:

(i) SEQUENZ CHARAKTERISTIKA:
(A) LÄNGE: 10 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 14:

Gln Lys Lys Asn Lys Arg Asn Thr Asn Arg
1               5                   10

(2) INFORMATION ZU SEQ ID NO: 15:

(i) SEQUENZ CHARAKTERISTIKA:
(A) LÄNGE: 15 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 15:

Asn Pro Lys Pro Gln Arg Lys Thr Lys Arg Asn Thr Asn Arg Arg
1               5                   10                  15

(2) INFORMATION ZU SEQ ID NO: 16:

(i) SEQUENZ CHARAKTERISTIKA:
(A) LÄNGE: 16 Aminoäuren
(B) ART: Aminoäure
(D) TOPOLOGIE: linear

(ii) ART DES MOLEKÜLS: Peptid

(xi) SEQUENZBESCHREIBUNG: SEQ ID NO: 16:

Pro Gln Asp Val Lys Phe Pro Gly Gly Gly Gln Ile Val Gly Gly Val
1               5                   10                  15

(2) INFORMATION ZU SEQ ID NO: 17:

(i) SEQUENZ CHARAKTERISTIKA:
(A) LÄNGE: 12 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 17:

Pro Gln Asp Val Lys Phe Pro Gly Gly Gly Gln Ile
1               5                   10

(2) INFORMATION ZU SEQ ID NO: 18:

(i) SEQUENZ CHARAKTERISTIKA:
(A) LÄNGE: 9 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 18:

Lys Phe Pro Gly Gly Gly Gln Ile Phe
1               5

(2) INFORMATION ZU SEQ ID NO: 19:

(i) SEQUENZ CHARAKTERISTIKA:
(A) LÄNGE: 12 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 19:

Gln Asp Val Lys Phe Pro Gly Gly Gly Gln Ile Val
1               5                   10

(2) INFORMATION ZU SEQ ID NO: 20:

(i) SEQUENZ CHARAKTERISTIKA:
(A) LÄNGE: 9 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 20:

Lys Phe Pro Gly Gly Gly Gln Ile Val
1               5

(2) INFORMATION ZU SEQ ID NO: 21:

(i) SEQUENZ CHARAKTERISTIKA:
(A) LÄNGE: 9 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 21:

Gln Asp Val Lys Phe Pro Gly Gly Gly
1               5

(2) INFORMATION ZU SEQ ID NO: 22:

(i) SEQUENZ CHARAKTERISTIKA:
(A) LÄNGE: 15 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 22:

Pro Arg Gly Ser Arg Pro Ser Trp Gly Pro Thr Asp Pro Arg Arg
1               5                   10                  15

(2) INFORMATION ZU SEQ ID NO: 23:

(i) SEQUENZ CHARAKTERISTIKA:
(A) LÄNGE: 47 Aminosäuren
(B) ART: Aminosäure
(D) TOPOLOGIE: linear
(ii) ART DES MOLEKÜLS: Peptid

(xi) SEQUENZBESCHREIBUNG: SEQ ID NO: 23:

Gln Leu Phe Thr Phe Ser Pro Arg Arg His Trp Thr Thr Gln Gly Cys
1               5                   10                  15

Asn Cys Ser Ile Tyr Pro Gly His Ile Thr Gly His Arg Met Ala Trp
            20                  25                  30

Asp Met Met Met Asn Trp Ser Pro Thr Thr Ala Leu Val Met Ala
        35                  40                  45

(2) INFORMATION ZU SEQ ID NO: 24:

(i) SEQUENZ CHARAKTERISTIKA:
(A) LÄNGE: 18 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 24:

Met Ala Trp Asp Met Met Met Asn Trp Ser Pro Thr Thr Ala Leu Val
1               5                   10                  15

Met Ala

(2) INFORMATION ZU SEQ ID NO: 25:

(i) SEQUENZ CHARAKTERISTIKA:
(A) LÄNGE: 45 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 25:

Arg Gly Pro Arg Leu Gly Val Arg Ala Thr Arg Lys Thr Ser Glu Arg
1               5                   10                  15

Ser Gln Pro Arg Gly Arg Arg Gln Pro Ile Pro Lys Ala Arg Arg Pro
            20                  25                  30

Glu Gly Arg Thr Trp Ala Gln Pro Gly Tyr Pro Trp Pro
        35                  40                  45

(2) INFORMATION ZU SEQ ID NO: 26:

(i) SEQUENZ CHARAKTERISTIKA:
(A) LÄNGE: 21 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 26:

Arg Gly Pro Arg Leu Gly Val Arg Ala Thr Arg Lys Thr Ser Glu Arg
1               5                   10                  15

Ser Gln Pro Arg Gly
            20

(2) INFORMATION ZU SEQ ID NO: 27:

(i) SEQUENZ CHARAKTERISTIKA:
(A) LÄNGE: 20 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 27:

Ser Gln Pro Arg Gly Arg Arg Gln Pro Ile Pro Lys Ala Arg Arg Pro
1               5                   10                  15

Glu Gly Arg Thr
            20

(2) INFORMATION ZU SEQ ID NO: 28:

(i) SEQUENZ CHARAKTERISTIKA:
(A) LÄNGE: 15 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 28:

Lys Ala Arg Arg Pro Glu Gly Arg Thr Trp Ala Gln Pro Gly Tyr
1               5                   10                  15

(2) INFORMATION ZU SEQ ID NO: 29:

(i) SEQUENZ CHARAKTERISTIKA:
(A) LÄNGE: 11 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 29:

Gln Lys Lys Ala Ala Arg Asn Thr Asn Arg Arg
1               5                   10

(2) INFORMATION ZU SEQ ID NO: 30:

(i) SEQUENZ CHARAKTERISTIKA:
(A) LÄNGE: 15 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 30:

His Trp Thr Thr Gln Gly Ser Asn Ser Ser Ile Tyr Pro Gly His
1               5                   10                  15

(2) INFORMATION ZU SEQ ID NO: 31:

(i) SEQUENZ CHARAKTERISTIKA:
(A) LÄNGE: 18 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 31:

Ser Ser Ile Tyr Pro Gly His Ile Thr Gly His Arg Met Ala Trp Asp
1               5                   10                  15

Met Met

(2) INFORMATION ZU SEQ ID NO: 32:

(i) SEQUENZ CHARAKTERISTIKA:
(A) LÄNGE: 16 Aminosäuren
(B) ART: Aminosäure
(D) TOPOLOGIE: linear

(ii) ART DES MOLEKÜLS: Peptid

(xi) SEQUENZBESCHREIBUNG: SEQ ID NO: 32:

Pro Glu Gly Arg Thr Trp Ala Gln Pro Gly Tyr Pro Trp Pro Leu Tyr
1               5                   10                  15